# EUROPEAN PATENT APPLICATION

(11) **EP 0 625 361 A2**
(43) Date of publication of application: **23.11.1994**
(21) Application number: 94201385.5
(22) Date of filing: 17.05.1994
(51) Int. Cl.: A61N 2/00

(54) **Very low frequency and very short duration sequential variable magnetic field generating system**

(30) Priority: 20.05.1993 ES 9301079
(71) Applicant: Palacios Remacha, Alberto, E-50005 Zaragoza (ES); Aviles Iriarte, Miguel Angel, E-50005 Zaragoza (ES)
(72) Inventor: Palacios Remacha, Alberto, E-50005 Zaragoza (ES); Aviles Iriarte, Miguel Angel, E-50005 Zaragoza (ES)
(74) Representative: Ungria Lopez, Javier

(57) **Abstract**

Very low frequency and very short duration sequential variable magnetic field generating system, of the type comprising a feed source and a frequency inlet, as well as a frequency synthesizer to obtain two signals of different duration, using one as a control signal to charge a condenser and a second one to discharge it, so that at the inlet of the two signals obtained with the synthesizer, there are respective triggers-separators (5) and (6) to activate both switches.

## Description

### OBJECT OF THE INVENTION

As is expressed in the title of the present specification, the following invention consists of a very low frequency and very short duration sequential variable magnetic field generating system, which is applicable essentially to inducing sleep, relaxation, reducing stress, etc., in a way similar to how it is achieved by autogenous training.

The proposed system is based on discharging a condenser through a very low resistance conductor, which will cause in connection with the latter, magnetic fields whose duration will be that of the discharge, and whose intensity will depend on the capacity of the condenser just like the discharge time for a specific conductor.

The conducting strip, proposed as a transducer, has the advantage that it is easy to place in any seat, mattress or place of rest in general, along the spine of the corresponding person.

### BACKGROUND OF THE INVENTION

When we want to induce electric currents in the human body, coils with a larger or smaller number of spirals are traditionally used, which makes them difficult to adapt to mattresses, backs of chairs, etc.

On the other hand, the type of electric pulses generated, to obtain the corresponding magnetic pulses, tend to be square waves. As the induced current, which is that which is going to be used, is obtained only during the variation of the field, this causes a loss of part of the useless energy in the area of the wave, in which the current is constant in the generator, which in turn makes the support material (conductor) heat up unnecessarily.

Patent of invention 538.259 claims an electronic system to generate variable frequency magnetic impulses from the frequency of the feed network, constituted of a circuit that comprises a feed source, a control block, an automatically controllable switch and a transducer.

The feed source, is formed by an alternating current-direct current convertor, that has means for generating oscillation with a standard or reference frequency, the value thereof being, equal or proportional to the characteristic frequency of the alternating current of the feed network.

The control block is formed by a pulse counter or frequency divider, which receives the reference pulses from the feed network, and each "n" pulse received lets one pass, in other words, it acts as a programmable or choosable "n" divider.

The signal obtained in said control block or frequency divider is applied to a bipolar power step, which is comprised of a monostable element or else by a simple coil and a power step, so that the cited dividing block, controls by means of said power step, the corresponding pulse that will start up the switch, so that the latter permits the passing of part of a positive or negative half-wave, of the feed network, which will be transformed by means of the transducer, into a magnetic field, whose direction will depend on the direction of the current while the pulse lasts.

The transducer, is comprised of a coil, whose shape and size will vary depending on the uses of the apparatus.

By means of this system pulses which are or are not alternating, synchronized with the frequency of the feed network, are obtained.

### DESCRIPTION OF THE INVENTION

The magnetic field generating system that is described in the present specification, tries to obtain very low frequency magnetic pulses by means of a conducting strip with a resistance very close to short circuit through which a condenser is discharged, causing fields lasting a very short time, which in turn will cause current pulses in any conductor or cnductors close by.

The system proposed in the present specification, of a very simple design, is based on the discharge of a condenser, through a very low resistance conductor, which will cause in connection with the latter, magnetic fields, whose duration will be that of the discharge and whose intensity will depend on the capacity of the condenser, just like the discharge time for a specific conductor.

On the other hand, the conducting strip, proposed as a transducer, is very easily placed in any seat, mattress, or place of rest in general, along the spine, inducing therein small electric potentials.

Upon the working frequency being very small, the system that we achieve with very small power components, gets intensities high enough to induce these small necessary potentials.

In order to complete the description that is going to be made hereinafter and to provide a better understanding of the features thereof, the present specification is accompanied by a set of drawings in whose figures the most significant details of the invention are represented in an illustrative and non-restrictive manner.

### BRIEF DESCRIPTION OF THE FIGURES

Figure 1 shows the block diagram corresponding to the feed source and to the inlet of a reference frequency, which in this case is that of the network.

Figures 2 and 3 show two block diagrams corresponding to a frequency synthesizer, the one of figure 3 being used to achieve two signals of a different duration.

Figure 4 shows a block diagram in which the two triggers-separators, activators of both solid state switches, are observed.

Figure 5 shows the conducting strip comprised of several in bridge conductors.

Figure 6 is another embodiment of the conducting strip adapted to the surface of a mattress.

### DESCRIPTION OF A PREFERRED EMBODIMENT

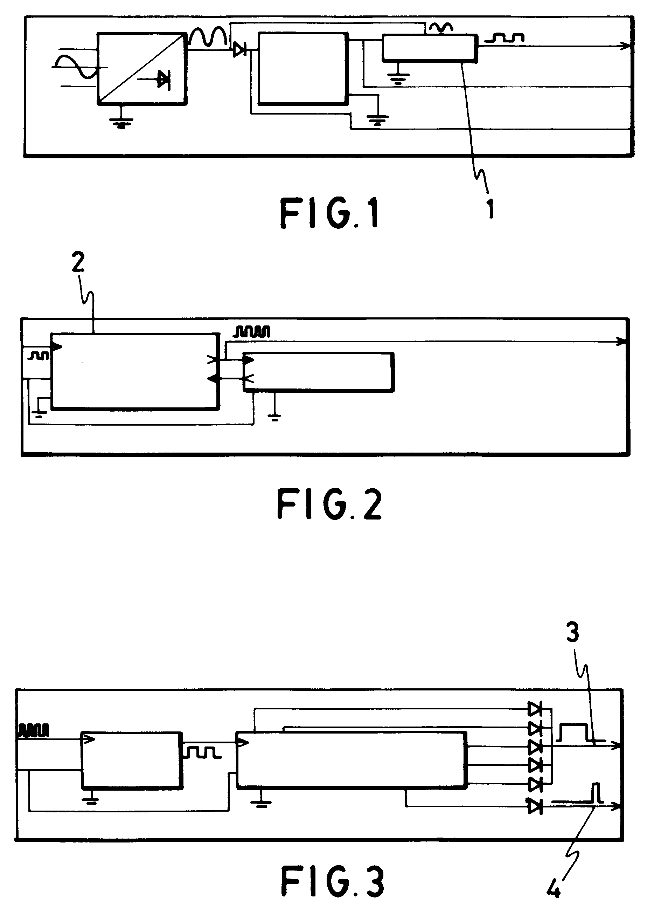

In view of the figures commented on and in accordance with the numbering used, we can see how the block diagram of figure 1 corresponds to the feed source and to the inlet of the reference frequency, which in our case is that of the network. It could be very well fed by a battery and the signal of the network by means of a probe that goes directly to the squarer (1.) The latter would pick up the network frequency due to proximity, and would introduce it into a CMOS logical gate, which would act as a detector and squarer, obtaining at the outlet the frequency of the network at the potential of the battery.

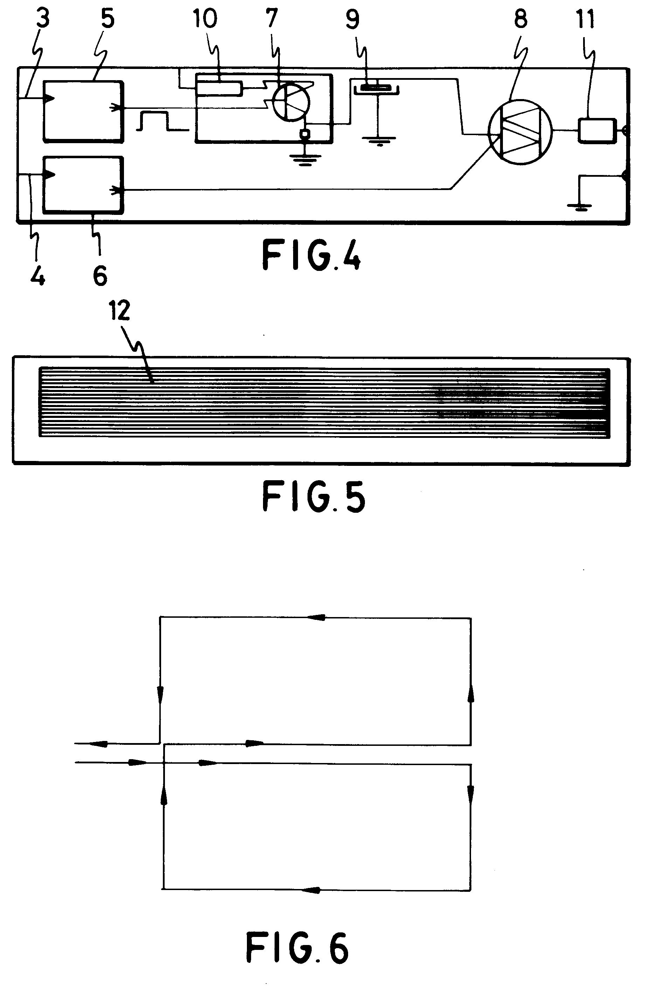

In figures 2 and 3 the block diagrams that correspond to a frequency synthesizer (2), which in turn is used (block 3) to attain two signals of a different duration (Sp1 3 and Sp2 4), have been represented. The first one will be used as a control signal to charge the condenser (C1) and the second one to discharge it.

These three block can be replaced by any feed source and by a programmable oscillator at the whose outlet we could obtain an asymmetrical wave and by means of an inverter we would achieve two signals, one direct one and the other inverting the latter similar to Sp1 and Sp2.

In figure 4 we can see how at inlet of the two signals (Sp1 3 and Sp2 4) there are two triggers-separators (5 and 6), whose task consists of activating both solid state switches (7) and (8).

When signal (Sp1) is positive, (Sp2) is zero, whereby the switch (7) is activated permitting the charge of the condenser (C1 9) through the resistor (R1 10.)

When the condenser (C1 9) is charged, the switch (7) passes to a non-conduction state (signal (Sp1) no longer acts, or if it is a thyristor or a triac, the switch (7) closes upon the condenser (C1 9) reaching its maximum charge.)

When the switch (7) closes, the switch (8) receives the signal (Sp2) and becomes active discharging the condenser (C1 9) through the resistor (R2 11) (to prevent the destruction of the switch and whose value is extremely low one or two Ohm approximately) and of the conducting strip (12) comprised of several in bridge conductors or a wide conductor.

The field obtained around the conducting strip (12) will act upon the part of the body that is close to it (generally the back), achieving the desired effect.

Hence, upon the work frequency being very low, the process described achieves, with very low power components, intensities high enough to induce the necessary small potentials.

The field obtained around the conducting strip will act on the body that is close to its (generally the back), achieving the desired effect.

As we have said above, the conducting strip can adapt perfectly to any surface, and due to the special features of the proposed system it is ideal for specific use, such as a mattress.

As in the manufacturing process of a mattress different factors to be taken into account take part, possibly the idealist form of the strip in this case is not the one indicated in figure 5, but rather avoiding stress that could break the conductors in a subsequent use of the mattress, the embodiment according to figure 6, with the advantage that it would be put in place in the last layer. The problem of the return of the cable would also be solved, and upon keeping the resistance very low, the triggering system would not vary at all.

## Claims

1. Very low frequency and very short duration sequential variable magnetic field generating system, of the type that is comprised of a feed source and a frequency inlet, as well as a frequency synthesizer, to obtain two signals of a different duration, one used as a control signal, to charge a condenser, and the second one to discharge it, essentially characterized in that at the input of the two signals (Sp1 and Sp2) obtained with the frequency synthesizer, respective triggers-separators (5 and 6) are found to activate both solid state switches (7) and (8), so that upon the signal (Sp1) being positive the signal (Sp2) is zero, and the switch (7) activates permitting the condenser C1 (9) to charge through the resistor R1 (10), while the switch (7) closes, the switch (8) receives the signal (Sp2) becoming active again, the condenser C1 (9) discharging through the resistor R2 (11) and the conducting strip (12), comprised of several in bridge conductors, which will create a field around it acting upon the person on the desired area.
